(19) Europäisches Patentamt
European Patent Office
Office européen des brevets

(11) **EP 4 324 457 A1**

(12) **EUROPEAN PATENT APPLICATION**

(43) Date of publication:
**21.02.2024 Bulletin 2024/08**

(21) Application number: **22190372.7**

(22) Date of filing: **15.08.2022**

(51) International Patent Classification (IPC):
***A61K 9/14*** *(2006.01)*     ***A61K 47/32*** *(2006.01)*
***A61K 47/12*** *(2006.01)*     ***A61K 47/38*** *(2006.01)*

(52) Cooperative Patent Classification (CPC):
**A61K 9/10; A61K 9/145; A61K 9/146; A61K 47/12;
A61K 47/32; A61K 47/38**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Bayer AG
51373 Leverkusen (DE)**

(72) Inventors:
• **Dobrowolski, Dr. Adrian
42899 Remscheid (DE)**
• **Kersten, Dr. Elisabeth
42105 Wuppertal (DE)**
• **Ostendorf, Dr. Michael
51375 Leverkusen (DE)**
• **Hoheisel, Dr. Werner
51061 Köln (DE)**
• **Nüboldt, Christoph
50969 Köln (DE)**

(74) Representative: **BIP Patents
c/o Bayer Intellectual Property GmbH
Alfred-Nobel-Straße 50
40789 Monheim am Rhein (DE)**

(54) **TRIPLE STABILIZER**

(57)     What is described herein relates to a compositions comprising
• a substance of a particle size between 1 $\mu$m and 100 $\mu$m and a solubility in water between 0.0001 g/l and 10 g/l
• at least one cellulose based polymer
• at least one surfactant, wherein the critical micelle concentration of the at least one surfactant is influenced by the at least one cellulose based polymer
• at least one steric stabilizer,
• wherein the steric stabilizer is present in an amount of steric stabilizer to at least one polymer in the range of between 10:1 and 1:10
• wherein the ratio of the at least one polymer and the at least one steric stabilizer together to the substance is between 2:1 to 1:2,
• and wherein the concentration of the substance of a particle size between 1$\mu$m and 100$\mu$m and a solubility in water between 0.0001 g/l and 10 g/l is in the range of 10.1 wt% to 15 wt%.

**Description**

Stabilizer systems are commonly used in the wet comminution of a poorly soluble active ingredients for the production of nanosuspensions, i.e. suspensions in which the active ingredient is present as particles in the submicron range (d90 <1 $\mu$m). Generally, such processes employ water-soluble polymers for steric stabilization and charged (ionic) surfactants for electrostatic stabilization of the active ingredient nanoparticles.

For economic reasons, a high concentration of active ingredients in nanosuspensions is preferred. In order to generate nanosuspensions with a high concentration of active ingredient a higher concentration of the stabilizing polymer has to be used as well. However, there is a concentration limitation for certain stabilizing polymers, either because their solubility in water is too low or because the viscosity becomes too high for comminution at higher polymer concentrations. For example, cellulose derivatives such as hydroxypropyl cellulose (HPC), which can be used as stabilizing polymer, lead to relatively high viscosities even at low concentrations, making processing of active ingredients with HPC as stabilizing polymer via comminution challenging or even impossible. Thus, until now the concentration of the active ingredient had to be reduced if HPC was used as stabilizing polymer, thereby also reducing the concentration of the stabilizing polymer until a viscosity was reached that allowed processing. Accordingly, active ingredients that are preferably stabilized with HPC could only be produced as a lower concentrated nanosuspension. Consequently, compared to higher concentrated suspensions, several batches had to be milled to produce a required amount of active ingredient nanoparticles. Therefore, there was a need to provide higher concentration suspensions of substances stabilized with HPC.

This need is met by providing a composition comprising

- a substance of a particle size between 1 $\mu$m and 100 $\mu$m and a solubility in water between 0.0001 g/l and 10 g/l
- at least one cellulose based polymer
- at least one surfactant, wherein the critical micelle concentration of the at least one surfactant is influenced by the at least one cellulose based polymer
- at least one steric stabilizer,
- wherein the steric stabilizer is present in an amount of steric stabilizer to at least one polymer in the range of between 10:1 and 1:10
- wherein the ratio of the at least one polymer and the at least one steric stabilizer together to the substance is between 2:1 to 1:2,
- and wherein the concentration of the substance of a particle size between 1 $\mu$m and 100 $\mu$m and a solubility in water between 0.0001 g/l and 10 g/l is in the range of 10,1 wt% to 15 wt%.

**BRIEF DESCRIPTION OF THE DRAWINGS**

FIG 1 shows that a composition comprising a triple stabilizer system has a lower viscosity than a composition comprising two stabilizers.

FIG 2 shows that via adding PVP K12 to a composition as described herein the viscosity was reduced until a plateau was reached. With further addition of PVP K12 the viscosity was increased again. The upper graph (triangles) represents a composition comprising 3 wt% HPC and 0.2 wt% SDS whereas the lower graph (circles) represents a composition comprising 2 wt% HPC and 0.2 wt% SDS.

FIG. 3 shows that low molecular weight steric stabilizer PVPK12 or high molecular weight steric stabilizer PVPK25 resulted in a reduction of viscosity. The upper graph (triangles) represents a composition comprising 2 wt% HPC, 0.2 wt% SDS and PVP K25 whereas the lower graph (circles) represents a composition comprising 2 wt% HPC, 0.2 wt% SDS and PVP K12.

FIG. 4 shows that addition of 1wt% to 4 wt% of PVPK12 causes reduction of viscosity of the HPMC-SDS solution.

FIG. 5 shows that if the polymer influences the critical micelle concentration of a surfactant than that surfactant is suitable for the composition provided herein.

FIG 6 shows the resulting particle size distribution after milling of composition A (10,5 wt% active ingredient)

FIG. 7 shows that high active ingredient compositions comprising nano-particles i.e. nanosuspensions are stable even when diluted.

FIG. 8 shows that with increasing HPC content in a system with only two stabilizers (SDS and HPC) the viscosity increases linearly.

DEFINITIONS

As used herein, the term "particle" refers to a solid, gel, or semisolid material having a relatively small size and characterized in that as said particles have a solubility of less than 10g/l in a suspension comprising water, matrix forming polymer(s) and optionally surfactants as determined e.g. by HPLC.

As used herein the term "micro-particle" which is used synonymously with microparticle refers to particles having an average particle size of between > 999 nm to 100µm.

Preferably the employed micro-particles have an average d90 of between 2µm and 50µm, more preferably between 2µm and 30µm, most preferably between 2µm and 20µm.

As used herein the term "nano" refers to particles having an average particle size - i.e. d50 of the distribution - of ≥ 10 nm to ≤ 999 nm.

Herein the nanoparticles have a particle size distribution of d90 < 2µm preferably < 1µm especially preferred < 500 nm and d50 < 1 µm preferably < 500 nm especially preferred < 200 nm. The particle size distribution can be determined by methods known in the art such as laser diffraction or Dynamic Light Scattering (DLS). The term d50 or d90 means that 50% or 90% of the total product volume consists of particles smaller than the given value for d50 or d90. The given particle size corresponds to the hydrodynamic particle size when determined by DLS (dynamic light scattering), laser diffraction or electron microscopy and for all methods an equivalent size for spheres is given and meant here. A person skilled in the art is capable of choosing technical equipment suitable for achieving a respective d50 or d90 value. For example it is clear to a skilled person that using an agitated ball mill will result in a smaller d50 and d90 values than using a planetary ball mill, especially if the agitated ball mill is cooled.

If Laser diffraction was used to determine the particle size said laser diffraction was performed in a Malvern Mastersizer 3000. Suspensions (nano- and micro-) were directly injected into the storage tank of Master sizer 3000 without addition of further excipients. The Mastersizer 3000 was equipped with a Hydro MV unit, where deionized water was provided to disperse and dilute the sample further. The dispersed sample was added to the Hydro MV unit until the obscuration reached 5%. The mixer of the Hydro MV unit was set to 2500 rpm. The first three measurements were performed with red and blue laser lights using the Mie light scattering model. Three measurements were performed without ultrasonic.

As used herein the term "active ingredient" refers to an agent, active ingredient, compound, substance, compositions, or mixtures thereof, that provides a pharmacological and/or agrochemical effect.

As used herein the term "critical micelle concentration" (CMC) refers to the minimum concentration of surfactants which is necessary to form a colloidal system, in particular micelles, in a self-assembly process. The critical micelle concentration can be determined as described in A. Fernández, N. Gonzalez, W. Iglesias, and L. Montenegro (1997) Determination of Critical Micelle Concentration of Some Surfactants by Three Techniques, J. Chemical Education, 74:1227-1231.)

As used herein the term "steric stabilizer" refers to a polymer or a non-ionic amphiphilic compound (surfactant) that sterically stabilizes the nano-particles.

## DETAILED DESCRIPTION

As specified above according to a first aspect what is described herein relates to a composition comprising

- a substance of a particle size between 1 µm and 100 µm and a solubility in water between 0.0001 g/l and 10 g/l
- at least one cellulose-based polymer
- at least one surfactant, wherein the critical micelle concentration of the at least one surfactant is influenced by the at least one cellulose-based polymer
- at least one steric stabilizer,
- wherein the steric stabilizer is present in an amount of steric stabilizer to at least one polymer in the range of between 10:1 and 1:10
- wherein the ratio of the at least one polymer and the at least one steric stabilizer together to the substance is between 2:1 to 1:2,
- and wherein the concentration of the substance of a particle size between 1 µm and 100 µm and a solubility in water between 0.0001 g/l and 10 g/l is in the range of 10,1 wt% to 15 wt%.

It was found that via adding the steric stabilizer the viscosity of the composition before and after wet comminution was reduced. This was surprising as it had been expected that adding a further steric stabilizer would further increase the viscosity. Therefore, via adding the steric stabilizer it became possible to increase concentration of the at least one cellulose-based polymer while still maintaining the viscosity in a range suitable for comminution/milling. Due to the higher polymer concentration also concentration of the substance present in form of nanoparticles could be increased. Thus, employing the steric stabilizer results in a more economic process since the comminution has to be repeated less times.

Viscosity can be measured for example via rotational viscosimeter, capillary viscosimeter, falling sphere viscosimeter or dip cup viscosimeter which all are established methods and well known to a person skilled in the art. An example for a rotational viscosimeter is the modular compact rheometer MCR102e by Anton Paar. For flow and viscosity curves measured via rotational viscometry, a shear rate range is specified, and the viscosity is measured as a function of the shear rate. In the case of the flow curve, the shear stress is usually shown on the y-axis and the shear rate on the x-axis; in the case of the viscosity curve, the viscosity is shown on the y-axis and the shear rate on the x-axis. (Rotational viscometry :: Anton Paar Wiki (anton-paar.com) )

In a preferred embodiment of the composition the substance of a particle size between 1 µm and 100 µm and a solubility in water between 0.0001 g/l and 10 g/l is an active ingredient.

A skilled person is aware of the fact that the composition described herein could also comprise more than one substance e.g. a mixture of two active ingredients. However, in practice this embodiment seems unlikely due to regulatory requirements regarding active ingredients.

Preferably, the cellulose-based polymer is selected from methylcellulose, hydroxymethylcellulose, hydroxyethylcellulose, hydroxypropylcellulose, hydroxybutylcellulose, hydroxyethylmethylcellulose, hydroxypropylmethylcellulose, carboxymethylcellulose, sodiumcarboxymethylcellulose, carboxymethylethylcellulose and carboxyalkylcellulose More preferably the cellulose based polymer is selected from hydroxymethylcellulose (HPMC) and hydroxypropylcellulose (HPC).

In an especially preferred embodiment HPC is used as cellulose based polymer

The surfactant can be selected from any surfactant (i.e. charged or non-charged) as long as the critical micelle concentration of the surfactant is influenced by the polymer. If this is the case than the surfactant is suitable.

When employing a charged surfactant the composition described herein also has beneficial effects for the production of re-dispersible powders as described in WO2021069350A1. To produce redispersible powders after drying of these nanosuspensions, it was shown in WO2021069350A1 that a drug to polymer ratio of ~1:1 is advantageous. By re-dispersible powders it is meant that these powders, with the addition of the at least equal amount of water removed during drying, form a suspension with a size distribution where again the d90 is < 1 $\mu$m. If the suspension has a higher concentration less water has to be evaporated during drying to obtain the desired amount of redispersible powder (WO2021069350A1).

Surfactants known to a skilled person include charged surfactant selected from acylamino acids (and salts thereof), such as: acylglutamates, for example sodium acylglutamate, di-TEA-palmitoylaspartate and sodium acaprylglutamate; acylpeptides, for example palmitoyl hydrolyzed milk protein, sodium cocoyl hydrolyzed soy protein and sodium/potassium cocoyl hydrolyzed collagen; Sarcosinates, for example, myristoyl sarcosine, TEA-lauroyl sarcosinate, sodium lauroyl sarcosinate and sodium cocoyl sarcosinate; Taurates, for example, sodium lauroyl taurate and sodium methyl cocoyl taurate; acyl lactylates, luroyl lactylate, caproyl lactylate, alaninates; carboxylic acids and derivatives, such as: carboxylic acids, for example lauric acid, aluminum stearate, magnesium alkanolate and zinc undecylenate; ester carboxylic acids, for example calcium stearoyl lactylate and sodium PEG lauramide carboxylate; Ether carboxylic acids, for example sodium laureth carboxylate and sodium PEG cocamide carboxylate; phosphoric acid esters and salts, such as DEA-oleth-phosphate and dilaureth-phosphate; sulfonic acids and salts, such as acyl-isethionates, e.g. sodium/ammonium cocoyl isethionate, alkyl aryl sulfonates, alkyl sulfonates, for example sodium coco monoglyceride sulfate, sodium C-olefin sulfonate, sodium lauryl sulfoacetate and magnesium PEG cocamide sulfate, sulfosuccinates, for example dioctyl sodium sulfosuccinate, disodium laureth sulfosuccinate, disodium lauryl sulfosuccinate and disodium undecylenamido MEA sulfosuccinate; as well as sulfuric acid esters, such as alkyl ether sulfate, for example sodium, ammonium, magnesium, MIPA, TIPA laureth sulfate, sodium myreth sulfate and sodium C-pareth sulfate, alkyl sulfates, for example sodium, ammonium and TEA lauryl sulfate,, alkylamines, alkylimidazoles, ethoxylated amines, quaternary surfactants, esterquats and amphoteric surfactants such as Acyl/dialkyl ethylenediamines, for example sodium acylamphoacetate, disodium acylamphodipropionate, disodium alkylamphodiacetate, sodium acylamphohydroxypropyl sultonate, disodium acylamphodiacetate and sodium acylamphopropionate, and N-alkylamino acids, for example, aminopropylalkylglutamide, alkylaminopropionic acid, natuium alkylimidodipropionate and lauroamphocarboxyglycinate.

In a preferred embodiment sodium dodecyl sulfate (SDS), sodium docusate (dioctyl sodium sulfosuccinate) and/or sodium oleate is used as surfactant

Examples of steric stabilizers are polyvinylpyrrolidone-vinylacetate-copolymer, polymethracrylatederivates, and polyvinylpyrrolidone (PVP). methylcellulose, hydroxymethylcellulose, hydroxyethylcellulose, hydroxypropylcellulose, hydroxybutylcellulose, hydroxyethylmethylcellulose, hydroxypropylmethylcellulose, carboxymethylcellulose, sodiumcarboxymethylcellulose, carboxymethylethylcellulose, carboxyalkylcellulose ester, starches, sodium carboxymethylamylopectin, chitosan, dextran sulfate sodium salt, alginic acid, alkali metal and ammonium salts of alginic acid, carrageenans, galactomannans, tragacanth, agar-agar, gum arabic, guar gum, xanthan gum, polyacrylic acid and its salts, polymethacrylic acid and its salts, polyvinyl alcohol, polyvinylpyrrolidone, polyethylene oxide, polypropylene oxide, copolymers of ethylene oxide and propylene oxide, N-vinylpyrrolidone-vinyl acetate copolymers or a mixture of at least two of the aforementioned polymers. Polyvinylpyrrolidones (especially K12 and K30 types) and N-vinylpyrrolidone-vinyl acetate copolymers are particularly preferred. Preferably, the polymer can be a copolymer of ethylene oxide and propylene oxide, in particular a poloxamer, or a polyvinylpyrrolidone, in particular PVPK30.

In a further preferred embodiment the steric stabilizer is characterized by a molecular weight between 2000 g/mol and 16.000g/mol preferably between 3000 g/mol and 8.000 g/mol and is termed a "low molecular weight steric stabilizer".

In a further preferred embodiment the steric stabilizer is characterized by a molecular weight between 18.000 g/mol and 50.000g/mol preferably between 22.000 g/mol and 44.000 g/mol and termed a "high molecular weight steric stabilizer".

In another preferred embodiment the composition comprises 0,01 wt% to 10 wt% low molecular weight steric stabilizer or 0,01 wt% to 5wt% high molecular weight steric stabilizer.

In a preferred embodiment polyvinylpyrrolidone is used as steric stabilizer.

In an especially preferred embodiment the low molecular weight steric stabilizer is PVP K12.

In a preferred embodiment of the composition the steric stabilizer is present in an amount of steric stabilizer to at least one polymer in the range of between 4:1 and 1:4.

In a further preferred embodiment of the composition the ratio of the at least one polymer and the at least one steric stabilizer together to the substance is between 2:1 to 1:2. In an especially preferred embodiment of the of the composition the ratio of the at least one polymer and the at least one steric stabilizer together to the substance is 1:1, as this ratio is optimal for the generation of re-dispersible powders.

In another preferred embodiment of the composition the concentration of the substance of a particle size between 1 $\mu$m and 100 $\mu$m in the range of 10,1 wt% to 12 wt%.

In another preferred embodiment of the composition the solubility of the substance of a particle size between 1 $\mu$m and 100 $\mu$m in water is in the range of between 0.001g/l and 1 g/l.

In another preferred embodiment the composition consist of at least one active ingredient of a particle size between 1$\mu$m and 100$\mu$m and a solubility in water between 0.001 g/l and 1 g/l, HPMC as cellulose based polymer, SDS as surfactant, PVP12 as low molecular weight steric stabilizer, wherein the steric stabilizer is present in an amount of steric stabilizer to at least one polymer in the range of between 4:1 and 1:4, wherein the ratio of the at least one polymer and the at least one steric stabilizer together to the substance is 1:1 and wherein the concentration of the active ingredient of a particle size between 1 $\mu$m and 100$\mu$m and a solubility in water between 0.001 g/l and 10 g/l is in the range of 10.1 wt% to 12 wt%.

In an especially preferred embodiment the composition consist of at least one active ingredient of a particle size between 1$\mu$m and 100$\mu$m and a solubility in water between 0.001 g/l and 1 g/1, HPC as cellulose based polymer, SDS as surfactant, PVP12 as low molecular weight steric stabilizer, wherein the steric stabilizer is present in an amount of steric stabilizer to at least one polymer in the range of between 4:1 and 1:4, wherein the ratio of the at least one polymer and the at least one steric stabilizer together to the substance is 1:1 and wherein the concentration of the active ingredient of a particle size between1$\mu$m and 100 $\mu$m and a solubility in water between 0.001 g/l and 10 g/l is in the range of 10.1 wt% to 12 wt%.

In a preferred embodiment the composition is further processed to form a re-dispersible powder as described in WO2021069350A1. In detail the composition in this case is further process via nano-grinding thereby providing a composition comprising nano-particles of the substance characterized by a nano-particle size of a d90 between 10 nm and 999 nm as measured by Dynamic Light Scattering (DLS), laser diffraction or electron microscopy.

Suitable methods of nano-grinding (also termed nano grinding, nanogrinding or nanomilling) can be selected from the group consisting of wet bead milling in stirred media mills, wet bead milling in planetary mills - especially suitable for small amounts - and high pressure homogenization. Alternatively, the application of high shear forces in aqueous suspensions like in a high pressure homogenization process or the application of high impact forces between the particles like in a microfluidizer can be used to produce nano-particles.

To a skilled person it is clear that if nano grinding is used for providing the seeding material (i.e. production of nanoscale crystals) said nano grinding can only be performed if the nano-particles are not completely dissolved. Hence the skilled person would choose a suitable temperature within the temperature range of between 253 K and 323 K, preferably between 273 K and 303 K for nano-grinding the nano-particles in the aqueous suspension.

Preferably, if nano-grinding is used, a preferred nano-grinding time is observed during grinding in order to achieve better redispersion of the dried powder containing nanoparticles. This preferred grinding time (t-preferred) is significantly longer than the grinding time that would typically be required to achieve the necessary particle size. Preferably said preferred grinding time (t-preferred) is at least 1.5 times t0, preferably it is at least 2 times t0 and particularly preferably at least 4 times t0. Here, t0 is considered to be the usual grinding time at which the d90 value of the particle size distribution is 1.5 times the d90 value reached after 12 hours and is referred to as d90(12h). In other words, this means that d90 (12h) is 2/3 of the d90 value at the usual grinding time t0. Grinding time is understood to mean the residence time of the suspension in the mill, which explicitly means that the possible residence time of the suspension in an optionally present holding tank is not counted towards the grinding time, i.e. towards the comminution time (tz).

Following nano-grinding the composition is spray dried according to step B) of the method described in WO 2021/069350 and to form a powder and optionally further process to form a tablet. This has the advantage that it can be stored more convenient.

It is especially preferred that of the composition is processed via nano-grinding and spray drying the ratio of the at least one polymer and the at least one steric stabilizer together to the substance is 1:1. Alternatively to nano-grinding the seeding material (i.e. the nano crystals) can be provided via high pressure homogenization, or antisolvent precipitation.

In another aspect what is described herein relates to a method for producing a composition comprising nano-particles starting from the composition described herein, wherein the method comprises the following steps:

- providing composition as described above comprising a substance of a particle size between 1 $\mu$m and 100$\mu$m and a solubility in water between 0.0001 g/l and 10 g/l, at least one cellulose based polymer, at least one surfactant and at least one steric stabilizer, wherein the at least one steric stabilizer is present in an amount of steric stabilizer to at least one cellulose based polymer in the range of between 10:1 and 1:10 wherein the ratio of the at least one cellulose based polymer and the at least one steric stabilizer together to the substance a particle size between 1$\mu$m and 1 nm is between 2:1 to 1:2, and wherein the concentration of the substance in the range of 10.1 wt% to 15 wt%.
- comminution of the composition for a time span until the particle size stays constant in a temperature range between 253K and 323 K thereby generating the composition comprising nano-particles.

In yet another aspect what is described herein relates to a composition comprising

- a substance of a particle size between 1 nm and 999 nm and a solubility in water between 0.0001 g/l and 10 g/l
- at least one cellulose based polymer
- at least one surfactant, wherein the critical micelle concentration of the at least one surfactant is influenced by the at least one cellulose-based polymer
- at least one steric stabilizer,
- wherein the steric stabilizer is present in an amount of steric stabilizer to at least one polymer in the range of between 10:1 and 1:10
- wherein the ratio of the at least one polymer and the at least one steric stabilizer together to the substance is between 2:1 to 1:2,
- and wherein the concentration of the substance of a particle size between 1$\mu$m and 100$\mu$m and a solubility in water between 0.0001 g/l and 10 g/l is in the range of 10.1 wt% to 15 wt%

for use as a medicament.

Said composition for use as a medicament is preferably a nano-suspension. Said composition is preferably manufactured via the method for producing a composition comprising nano-particles described above.

EXAMPLES

1. Viscosity of a triple stabilizer system compared to a composition comprising two stabilizers

In this example two compositions were compared. Composition A comprised as substance an active ingredient (10.5 wt%, here Rivaroxaban), as the at least one cellulose-based polymer hydroxypropylcellulose (HPC) 2 wt%, as the at least one surfactant a charged surfactant here SDS (0.2% SDS) and as the at least one steric stabilizer a low molecular weight steric stabilizer PVP K12 (8 wt%).

Composition B comprised 10.5 wt % active ingredient (here Rivaroxaban), 10 wt % HPC and 0.2 wt % SDS. The components of both compositions were stirred on a magnetic stirrer at room temperature.

Viscosity was measured with rotational viscometry with a modular compact rheometer (MCR) 302e (Anton Paar). A concentric cylinder geometry was used. Shear rate was varied logarithmic from 0.1 1/s to 1000 1/s. 6 measurement points per decade were used. The measurement time was varied form 10s for a shear rate of 0.1 1/s and Is for 1000 1/s. Shear rate vs. Shear stress was plotted. The slope described the viscosity. The shear rate independent viscosity was evaluated, and the middle value was used.

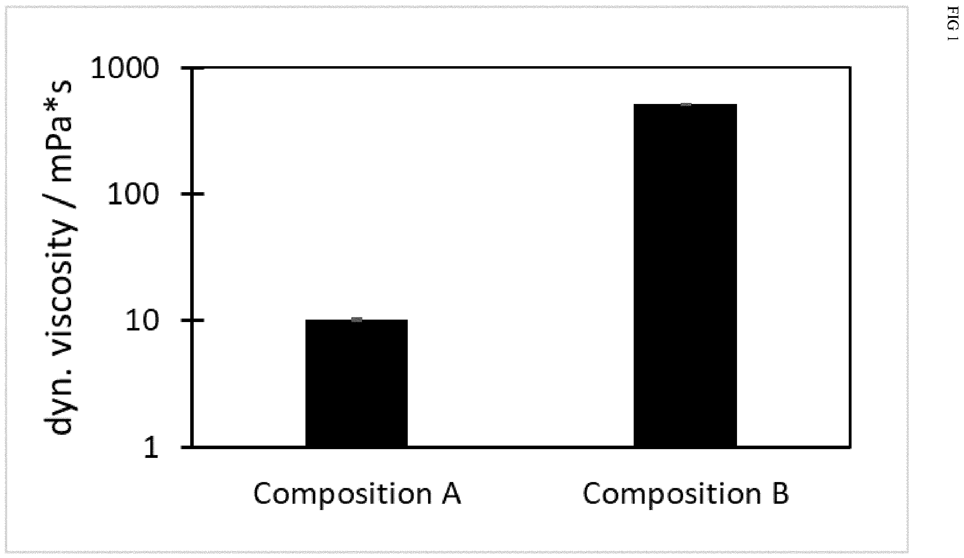

As depicted in Fig. 1 it was found that composition A (API-HPC-PVPK12-SDS) had a lower viscosity than composition B (API-HPC-SDS).

2. High active ingredient nanosuspension

Furthermore, the viscosity of composition B was so high, that it was not possible to separate the milling beads from the high viscous composition B during wet milling. Hence it was not practical to process composition B into nanosuspension via wet milling. The processing of composition A via wet milling on the other hand was unproblematic and resulted in a stable high active ingredient nanosuspension. Separation of grinding beads from the nanosuspension was possible.

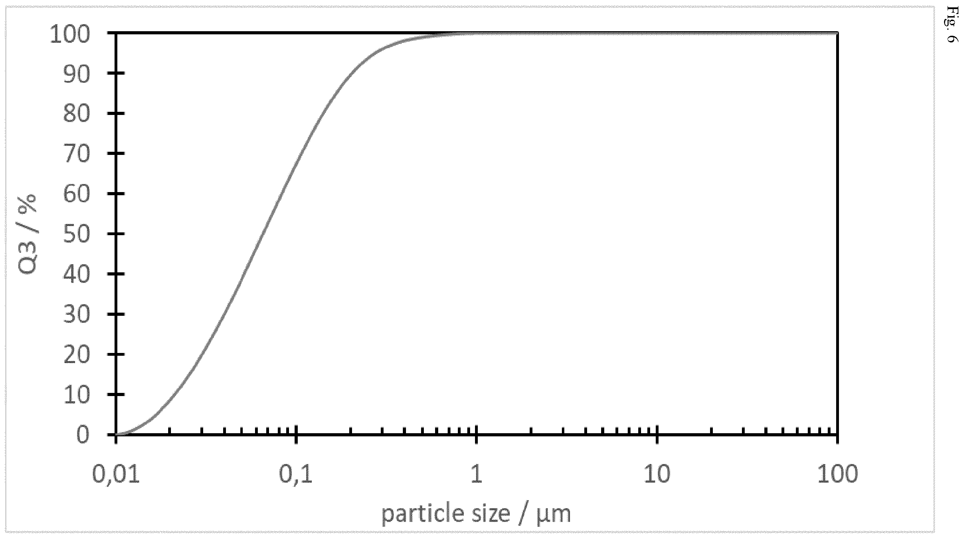

To evaluate whether a high active ingredient nano-suspension could also be generated using only a charged surfactant here SDS (0.2% SDS) and a low molecular weight steric stabilizer PVP K12 (8 wt%) composition C comprising 10.1 wt% API (here Rivaroxaban), 10wt% PVPK12 and 0.2 wt% SDS was produced and processed into a nanosuspension. The resulting suspension was instable after dilution and therefore unsuitable for further applications (Data not shown). Fig. 6 shows the resulting particle size distribution after milling of composition A (10.5 wt% active ingredient). Milling conditions were used as described in example 8.

The results of composition A were repeated with two other active ingredients (data not shown)

3. Influence of steric stabilizer (PVP) concentration

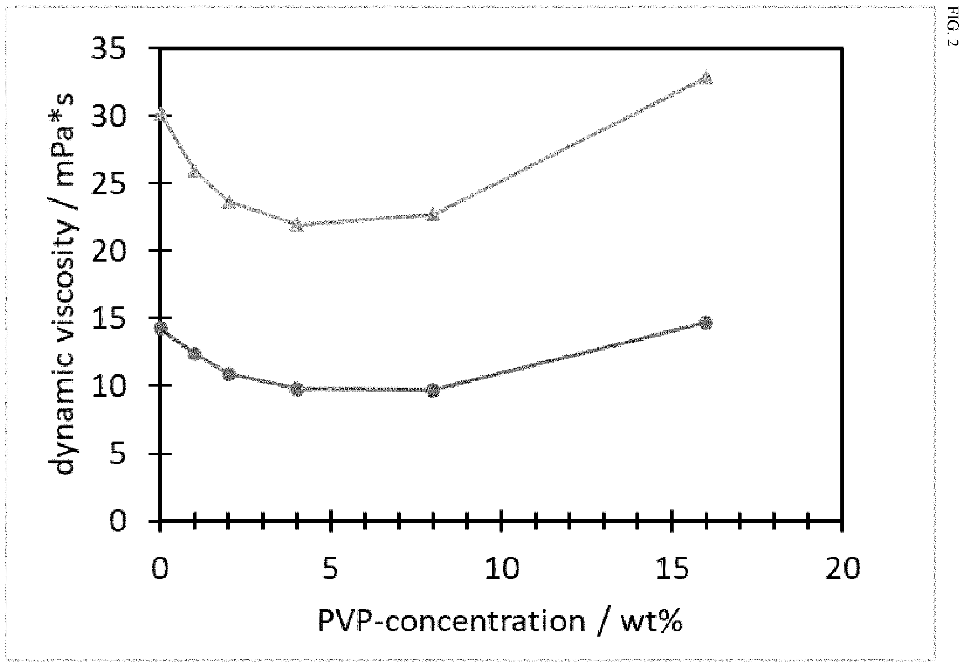

Moreover, it was found that with increasing PVP K12 content the viscosity was reduced until a plateau was reached. With further addition of PVP K12 the viscosity increased again (Fig. 2). Viscosity was measured as described in example 1.

### 4. Influence of steric stabilizer other than PVP 12

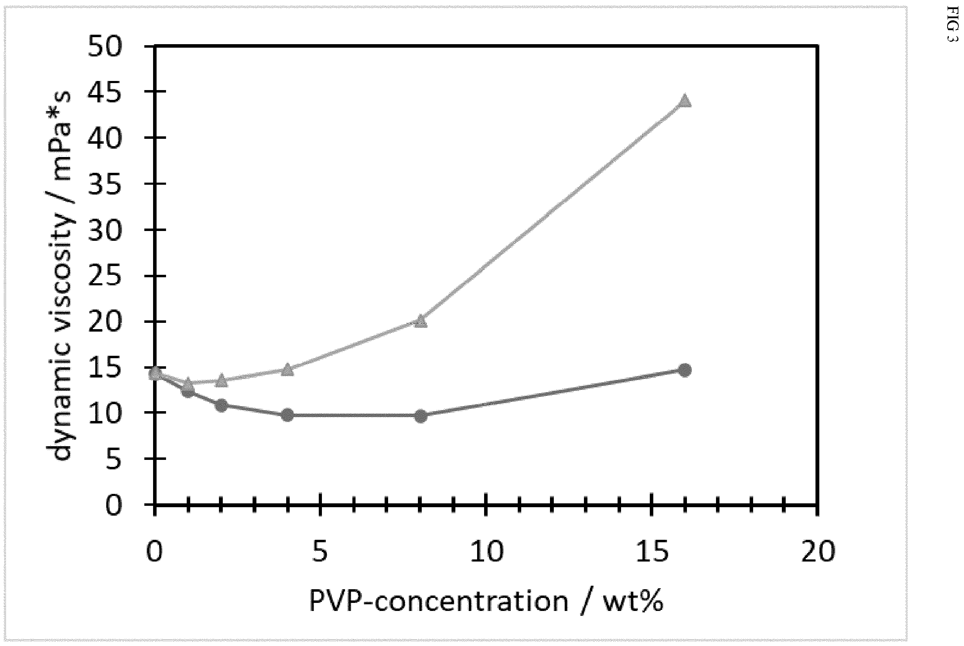

In this example HPC-SDS solutions (2wt% and 0.2 wt%) with varied amount of PVPK12 or PVPK25 were produced for a viscosity study. SDS and HPC content were kept constant (2 wt% HPC and 0.2 wt% SDS) and the PVPK12/PVPK25 content was varied. Fig 3 shows that low molecular weight PVP - here PVP K12 - was suitable for viscosity reduction of a HPC-SDS solution at least up to a value of 8 wt%. Also, addition of the high molecular weight steric stabilizer PVPK25 resulted in a reduction of viscosity if added up to 5wt%. Viscosity was measured as described in example 1.

### 5. Influence of cellulose-based polymer other than HPC

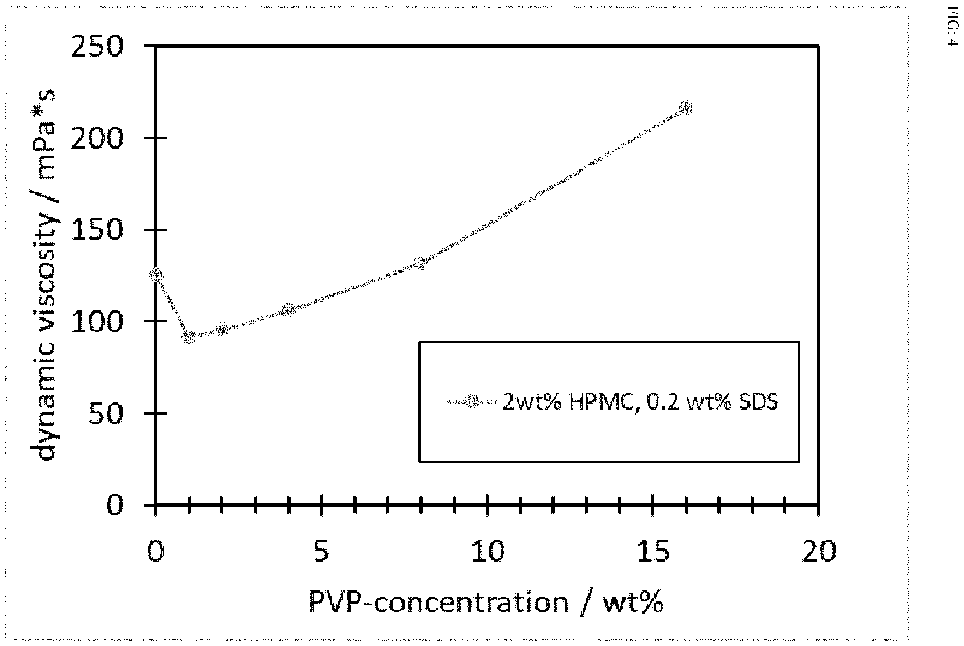

In this example HPC was replaced by HPMC to produce Cellulose-SDS solutions with different amount of PVPK12 for a viscosity study. SDS (0,2wt%) and HPMC (2wt%) content were kept constant and the PVPK12 content was varied in the range of 0-15 wt%. Fig 4 shows that addition of 1wt% to 4 wt% of PVPK12 still cause reduction of viscosity of the HPMC-SDS solution. Viscosity was measured as described in example 1.

### 6. Influence of surfactants other than SDS

In this example HPC-PVP solutions with different surfactants Na-Docusate (DOSS), Na-Deoxycholate and SDS were produced. HPC concentration was constant at 2wt%, surfactant concentration was constant at 0.2wt%. PVPK12 concentration was varied from 0 to 16 wt%. Viscosity was measured as described in example 1. For better comparability of samples, the relative viscosity change was calculated and plotted against PVPK12 concentration.

$$\Delta\eta_{rel} = \frac{\eta_n - \eta_0}{\eta_0}$$

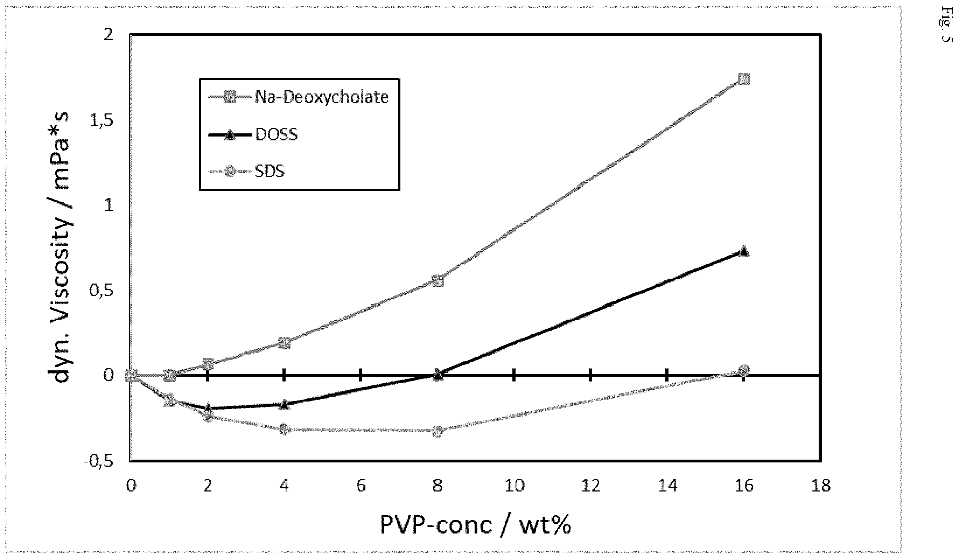

was used for calculation of relative viscosity change. $\eta_0$ represents the viscosity of the respective cellulose derivative surfactant solution without PVP and $\eta_n$ the respective viscosity of the cellulose derivative surfactant solutions after PVP has been added. If $\eta_n = \eta_0$ the resulting relative viscosity change is zero. Fig 5 shows the impact of surfactant on the viscosity reduction induced by PVPK12 addition. SDS (circles, lowest curve) has the strongest impact followed by DOSS (middle curve, triangles). Na-Deoxycholate (top curve, squares) containing solutions do not show a reduction of viscosity when PVPK12 is added. It is literature known that Na-Deoxycholate does not have a good interaction with PVP or cellulose derivates (Majhi, 2000, physicochemical investigations on the interaction of surfactants and salts with polyvinylpyrrolidone in aqueous medium). In other words the data shows that the critical micelle concentration of the surfactant was not influenced by the cellulose derivates (i.e. the polymer) or the steric stabilizer. Hence in this example the polymer-steric stabilizer solution only influenced the critical micelle concentration of the surfactants Na-Docusate (DOSS) and SDS. Therefore Na-Docusate (DOSS) and SDS were suitable surfactants.

### 7. Influence of increasing HPC in a system with two stabilizers

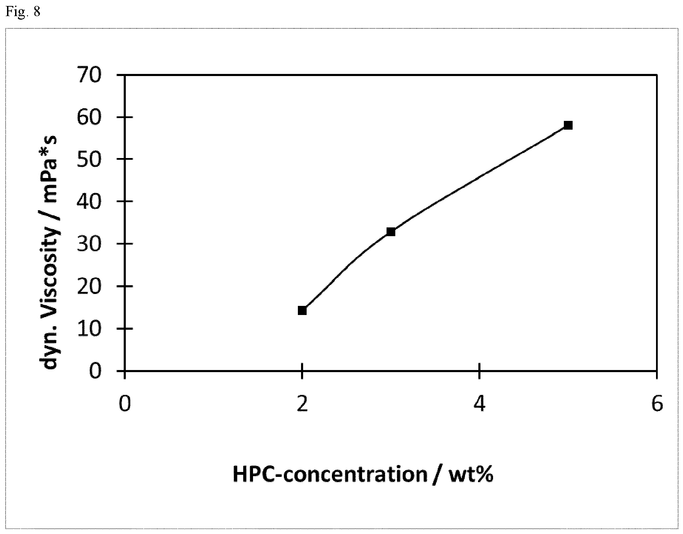

In this example water-based HPC-SDS solutions (i.e. solutions with only two staiblizers) were prepared with constant SDS-concentration (0.2wt%) and varied HPC-concentration. Fig. 8 shows that with increasing HPC content the viscosity increases linearly. Viscosity was measured as described in example 1.

### 8. Dilution experiments

In order to show that the high active ingredient compositions are stable even when diluted as it would be the case if an active ingredient is applied to a subject, different settings were compared In first step stabilizer solutions were prepared

   (1) 10 wt% PVPK12, 0.2 wt% SDS,
   (2) 8 wt% PVPK12, 2 wt% HPC and 0.2 wt% SDS and
   (3) 2 wt% HPC and 0.2 wt% SDS.

After that API-microparticles were dispersed inside the prepared stabilizer solution. The result were three microsuspensions:

   (1) active ingredient (Rivaroxaban) 10 wt% ,10 wt% PVPK12, 0.2 wt% SDS
   (2) active ingredient (Rivaroxaban) 10 wt% 8 wt% PVPK12, 2 wt% HPC and 0.2 wt% SDS
   (3) active ingredient (Rivaroxaban) 2 wt% 2 wt% HPC and 0.2 wt% SDS

These microsuspensions were the starting material for the preparation of nanosuspensions via stirred media milling. In the next step nano-suspensions were prepared from each of the three microsuspensions via stirred media milling in picoliq by hosokawa alpine. All suspensions were processed at 35°C with 2500-3000 rpm in 90 ml milling chamber until a constant particle size was reached. 80% of milling chamber volume was filled with 0.4-0.6 mm ZrO$_2$ grinding beads by silibeads.

The dilution of the nano-suspensions was carried out inside the storage tank of Mastersizer 3000. The systems 1.1, 2 and 3 were diluted in 100 ml distilled water at room temperature and constant stirrer speed of 1750 rpm. System 1.2 was diluted with PVPK12 and 0.2 wt% SDS instead of distilled water inside the storage tank of mastersizer 3000. At time point 0, all systems were diluted.

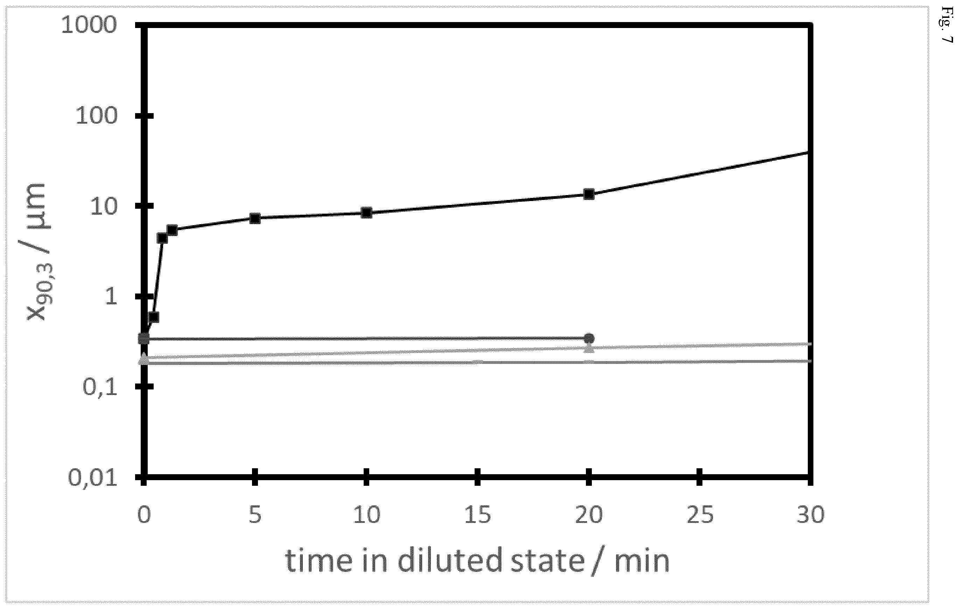

The results are depicted in Fig. 7. The figure shows the $x_{90,3}$ over time i.e. the 90 % of all particles had the depicted size or were smaller as measured by laser diffraction. The top graph (squares) ("system 1.1) represents an active ingredient (Rivaroxaban) 10 wt% nano-suspension which was stabilized with 10 wt% PVPK12 and 0.2 wt% SDS and measured at room temperature in 100 ml distilled water in mastersizer 3000 laser diffractometer by malvern. The top middle graph (circles) ("system 1.2") represents the same suspension but measured in stabilizer solution containing 10wt% PVPK12 and 0.2 wt% SDS instead of distilled water inside the storage tank of Mastersizer 3000. The top bottom graph (triangles) ("system 2") represents an active ingredient nanosuspension (Rivaroxaban) (10 wt%) which was stabilized with 8 wt% PVPK12, 2 wt% HPC and 0.2 wt% SDS. The bottom graph (no marking) ("system 3") represents an active ingredient (Rivaroxaban) 2 wt% nanosuspension which was stabilized with 2 wt% HPC and 0.2 wt% SDS and measured in 100ml distilled water at room temperature in mastersizer 3000 laser diffractometer by malvern. Hence in all cases the SDS concentration (0.2 wt%) was the same.

It was found that the dilution of system 1.1 inside water resulted in an instable composition since the nanoparticles agglomerated and formed larger particles in case of PVP stabilized nanosuspension.

Dilution with PVP in water (system 1.2) did not have this effect because the PVP in solution at the concentration used herein in the dilution medium ensured the stability of the diluted nanoparticles

System 3 did not show any agglomeration during the measurement time. However, it contained only 2wt% active ingredient.

Also, system 2 did not show any agglomeration, even though instead of 2 wt% (system 3), 10wt% API was used. Hence the triple stabilizer system ensured stability during dilution. Hence it can be concluded that a triple stabilizer system will also stabilize the active ingredient nano-particles under physiological conditions i.e. during dilution in the gastro-intestinal tract.

**Claims**

1. A compositions comprising

    • a substance of a particle size between 1 $\mu$m and 100 $\mu$m and a solubility in water between 0.0001 g/l and 10 g/l
    • at least one cellulose based polymer
    • at least one surfactant, wherein the critical micelle concentration of the at least one surfactant is influenced by the at least one cellulose based polymer
    • at least one steric stabilizer,
    • wherein the steric stabilizer is present in an amount of steric stabilizer to at least one polymer in the range of between 10:1 and 1:10
    • wherein the ratio of the at least one polymer and the at least one steric stabilizer together to the substance is between 2:1 to 1:2,

    • and wherein the concentration of the substance of a particle size between 1 $\mu$m and 100 $\mu$m and a solubility in water between 0.0001 g/l and 10 g/l is in the range of 10.1 wt% to 15 wt%.

2. Composition according to claim 1 wherein the composition the substance of a particle size between 1 $\mu$m and 100 $\mu$m and a solubility in water between 0.0001 g/l and 10 g/l is an active ingredient.

3. Composition according to claim 1 wherein the cellulose based polymer is hydroxypropylcellulose HPC.

4. Composition according to claim 1 wherein the surfactant is selected form the group consisting of sodium dodecyl sulfate (SDS), sodium docusate (dioctyl sodium sulfosuccinate) or sodium oleate.

5. Composition according to claim 1, wherein the composition comprises 0.01 wt% to 10 wt% low molecular weight steric stabilizer or 0.01 wt% to 5wt% high molecular weight steric stabilizer.

6. Composition according to claim wherein polyvinylpyrrolidone is used as steric stabilizer.

7. Composition according to claim 6, wherein the low molecular weight steric stabilizer is PVP K12.

8. Composition according to claim 1 wherein the steric stabilizer is present in an amount of steric stabilizer to at least one polymer in the range of between 4:1 and 1:4 .

9. Composition according to claim 1 wherein the ratio of the at least one polymer and the at least one steric stabilizer together to the substance of a particle size between 1 $\mu$m and 100 $\mu$m and a solubility in water between 0.0001 g/l and 10 g/l is between 2:1 to 1:2.

10. Method for producing a composition comprising nano-particles with the following steps:

    • providing a composition according to claim 1 comprising a substance of a particle size between 1 $\mu$m and 100 $\mu$m and a solubility in water between 0.0001 g/l and 10 g/l, at least one cellulose based polymer, at least one surfactant and at least one steric stabilizer, wherein the at least one steric stabilizer is present in an amount of steric stabilizer to at least one cellulose based polymer in the range of between 10:1 and 1:10 wherein the ratio of the at least one cellulose based polymer and the at least one steric stabilizer together to the substance a particle size between 1 $\mu$m and 1 nm is between 2:1 to 1:2, and wherein the concentration of the substance in the range of 10.1 wt% to 15 wt%.
 • comminution of the composition for a time span until the particle size stays constant in a temperature range between 253K and 323 K) thereby generating the composition comprising nanoparticles.

11. A composition comprising

 • a substance of a particle size between 1 nm and 999 nm and a solubility in water between 0.0001 g/l and 10 g/l
 • at least one cellulose based polymer
 • at least one surfactant, wherein the critical micelle concentration of the at least one surfactant is influenced by the at least one cellulose based polymer
 • at least one steric stabilizer,
 • wherein the steric stabilizer is present in an amount of steric stabilizer to at least one polymer in the range of between 10:1 and 1:10
 • wherein the ratio of the at least one polymer and the at least one steric stabilizer together to the substance is between 2:1 to 1:2,
 • and wherein the concentration of the substance of a particle size between 1$\mu$m and 100$\mu$m and a solubility in water between 0.0001 g/l and 10 g/l is in the range of 10.1 wt% to 15 wt%.

12. A composition comprising

 • a substance of a particle size between 1 nm and 999 nm and a solubility in water between 0.0001 g/l and 10 g/l
 • at least one cellulose based polymer
 • at least one surfactant, wherein the critical micelle concentration of the at least one surfactant is influenced by the at least one cellulose based polymer
 • at least one steric stabilizer,
 • wherein the steric stabilizer is present in an amount of steric stabilizer to at least one polymer in the range of between 10:1 and 1:10
 • wherein the ratio of the at least one polymer and the at least one steric stabilizer together to the substance is between 2:1 to 1:2,
 • and wherein the concentration of the substance of a particle size between 1$\mu$m and 100$\mu$m and a solubility in water between 0.0001 g/l and 10 g/l is in the range of 10.1 wt% to 15 wt%

for use as a medicament.

13. Composition according to claim 11 or claim 12 manufactured according to claim 10.

FIG 1

EP 4 324 457 A1

FIG. 2

FIG 3

FIG: 4

Fig. 5

Fig. 6

Fig. 7

Fig. 8

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/156223 A1 (ADVERIO PHARMA GMBH [DE]) 12 August 2021 (2021-08-12) * examples * * claims * | 1-13 | INV. A61K9/14 A61K47/32 A61K47/12 A61K47/38 |
| X | CERDEIRA ANA M ET AL: "Formulation and drying of miconazole and itraconazole nanosuspensions", INTERNATIONAL JOURNAL OF PHARMACEUTICS, ELSEVIER, NL, vol. 443, no. 1, 4 January 2013 (2013-01-04), pages 209-220, XP028979329, ISSN: 0378-5173, DOI: 10.1016/J.IJPHARM.2012.11.044 * page 212, right-hand column, last paragraph - page 213, left-hand column, paragraph 1 * * figure 2 * * page 217, left-hand column, last paragraph * | 1-13 | |
| A | BITTERLICH A ET AL: "Process parameter dependent growth phenomena of naproxen nanosuspension manufactured by wet media milling", EUROPEAN JOURNAL OF PHARMACEUTICS AND BIOPHARMACEUTICS, ELSEVIER SCIENCE PUBLISHERS B.V., AMSTERDAM, NL, vol. 92, 9 March 2015 (2015-03-09), pages 171-179, XP029226830, ISSN: 0939-6411, DOI: 10.1016/J.EJPB.2015.02.031 * table 1 * * page 177, right-hand column, last paragraph * | 1-13 | TECHNICAL FIELDS SEARCHED (IPC) A61K |

The present search report has been drawn up for all claims

| Place of search | Date of completion of the search | Examiner |
|---|---|---|
| Munich | 5 January 2023 | S. von Eggelkraut-G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 0372

This annex lists the patent family members relating to the patent documents cited in the above-mentioned European search report.
The members are as contained in the European Patent Office EDP file on
The European Patent Office is in no way liable for these particulars which are merely given for the purpose of information.

05-01-2023

| Patent document cited in search report | | Publication date | Patent family member(s) | | Publication date |
|---|---|---|---|---|---|
| WO 2021156223 | A1 | 12-08-2021 | CA | 3169671 A1 | 12-08-2021 |
| | | | CN | 115175666 A | 11-10-2022 |
| | | | EP | 4099987 A1 | 14-12-2022 |
| | | | WO | 2021156223 A1 | 12-08-2021 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021069350 A1

- WO 2021069350 A

**Non-patent literature cited in the description**

- **A. FERNÁNDEZ ; N. GONZALEZ ; W. IGLESIAS ; L. MONTENEGRO.** Determination of Critical Micelle Concentration of Some Surfactants by Three Techniques. *J. Chemical Education,* 1997, vol. 74, 1227-1231